Europäisches Patentamt

European Patent Office

Office européen des brevets

(11) Publication number: **0 106 395**
**A1**

# EUROPEAN PATENT APPLICATION

(21) Application number: 83201387.4

(22) Date of filing: 29.09.83

(51) Int. Cl.³: **A 61 N 5/06**
**F 21 S 3/12**

(30) Priority: 05.10.82 NL 8203861

(43) Date of publication of application:
25.04.84 Bulletin 84/17

(84) Designated Contracting States:
AT DE FR GB IT NL SE

(71) Applicant: N.V. Philips' Gloeilampenfabrieken
Groenewoudseweg 1
NL-5621 BA Eindhoven(NL)

(72) Inventor: De Jong, Willem Errit
c/o INT. OCTROOIBUREAU B.V. Prof. Holstlaan 6
NL-5656 AA Eindhoven(NL)

(72) Inventor: Mast, Jan
c/o INT. OCTROOIBUREAU B.V. Prof. Holstlaan 6
NL-5656 AA Eindhoven(NL)

(72) Inventor: Pepall, Lynford Stephen
c/o INT. OCTROOIBUREAU B.V. Prof. Holstlaan 6
NL-5656 AA Eindhoven(NL)

(74) Representative: Dusseldorp, Jan Charles et al,
INTERNATIONAAL OCTROOIBUREAU B.V. Prof.
Holstlaan 6
NL-5656 AA Eindhoven(NL)

(54) Irradiation apparatus.

(57) The invention relates to a displaceable total body irradiation apparatus suitable for use in the domestic field. The apparatus is provided with four reflectors (8) which are curved according to a parabola with a parameter $p$ and whose focal lines extend at a certain relative distance parallel to each other. The parameter $p$ has a value between 70 mm and 30 mm and the depth of each reflector (8) is at least $p$ mm.

The irradiation field (12) irradiated by means of the aparatus has a major axis (m') parallel to the focal lines of the reflectors (8).

EP 0 106 395 A1

./...

Croydon Printing Company Ltd.

FIG.1

0106395
15-2-1983

Irradiation apparatus.

The invention relates to irradiation apparatus suitable for irradiation of the human body and provided with a housing having two or more elongate reflectors which are curved according to a parabola with a parameter $\underline{p}$, whose focal lines extend at a certain relative distance parallel to each other and whose directrix planes enclose obtuse angles with each other of at least $160^{\circ}$ and at most $200^{\circ}$ whilst each reflector is provided with one or more lamp holders suitable for receiving a high-pressure discharge lamp with the discharge path being substantially along-the focal line.

In this description parabola is to be understood as the focus of points·whose distance from a focal point F equals their distance from a directrix L. The distance between the focal point F and the associated directrix L is denoted as the parameter $\underline{p}$.

Herein an elongate reflector is to be understood as a reflector which consists of a large number of congruent and connecting segments of parabola whose focal points form a common focal line and whose directrices form a common directrix plane.

An irradiation apparatus of the kind mentioned in the preamble is known from the German Offenlegungsschrift 1,764,608. In the known apparatus, each reflector is suitable for receiving several high-pressure discharge lamps. In this manner, during irradiation a comparatively large surface with substantially constant radiation intensity is obtained, which is suitable, for example, for a so-called total body irradiation. However, in the present-day lamps this leads to compararively large dimensions of each reflector and hence of the irradiation apparatus as a whole. Moreover, this leads to a comparatively heavy apparatus. This is less desirable when the apparatus is used in the domestic field.

The term "total body irradiation" is to be understood to mean herein an irradiation covering the whole body, which means in practice that the field of irradiation has a maximum length of at least 1.70 m and a maximum width of at least 0.60 m, whilst the term "field of irradiation" is to be understood in this description to mean the region which is enclosed by the points at which the radiation intensity is half the maximum intensity within this region.

The invention has for its object to provide a means by which the irradiation apparatus is rendered more suitable for use in the domestic field. An irradiation apparatus of the kind mentioned in the preamble is therefore characterized in that each reflector is suitable for receiving only one lamp and in that for each reflector the parameter $p$ lies between the limits of 70 mm and 30 mm and the depth of each reflector is at least $p$ mm.

In this description depth of the reflector is to be understood as the largest distance between the reflector and the emanation surface belonging to the reflector.

Due to this measure, it is possible to keep the dimensions and hence the weight of the irradiation apparatus comparatively small. It is a surprise to find that with an irradiation apparatus according to the invention, during irradiation a total body irradiation field of acceptable intensity is obtained. The reflectors may then either have a smooth surface or be composed of facets.

The advantage of comparatively small dimensions and a comparatively small weight can be explained as follows. When irradiation apparatus are used in the domestic field, the factor of the space available plays an important part because this space is mostly limited and the availability further has a non-permanent character. Due to the comparatively small dimensions and the comparatively small weight, an apparatus is obtained which can be displaced in a comparatively simple manner. This renders it possible to store the apparatus, for example, in a cupboard or a storage room, when it is not in use. During operation of the apparatus, the space to be used for the irradation can be chosen arbitrarily due to the simple displaceability.

A displaceable irradiation apparatus is known per se from the German Offenlegungsschrift 2, 827,802. A so-called total body irradiation cannot be realized , however, with this known apparatus. A displaceable irradiation apparatus is also known from the German Offenlegungsschrift 2,801,552. In this case, a pointed irradiation is concerned instead of a total body irradiation. Total body irradiation is mainly suitable for cosmetic purposes, which irradiation is preferably carried out with so-called UV-A radiation (315 nm - 400 nm). Under normal conditions, this radiation results in tanning of the skin. The degree of tanning then depends inter alia upon both the radiation intensity and the radiation time. Differences in the degree of tanning due to differences in intensity at ι the area of the field of irradiation prove to be acceptable as long as the intensity ratios remain limited to a factor 2.

The form of the parabola according to which the reflecters are curved, proves to have a predominant influence on the dimensions of the field of irradiation. Thus, a parameter p of the parabola larger than 70 mm with the same degree of concentration will lead to an excessively wide beam. In this case, a parameter p smaller than 30 mm leads to an excessively narrow beam of the radiation. On the other hand, a decrease of the depth of each reflector will reduce the degree of concentration of the radiation and hence the beam limitation, as a result of which with the desired field dimensions the maximum radiation intensity to be attained at the area of the field decreases to values which are unacceptable for practical use.

In a preferred embodiment of an irradiation apparatus according to the invention, the reflectors consist of two groups so that each group has a common directrix plane and that the two directrix planes enclose on the sides facing the focal lines an obtuse angle of at least

$165^{\circ}$ and at most $180^{\circ}$ with each other. Advantageously, a sharper beam limitation of the radiation is thus attained, which results _inter alia_ in that the intensity at the area of the field of irradiation is at maximum over approximately 8% of its surface.

In an advantageous embodiment of an irradiation apparatus according to the invention, the depth of each reflector is at most $3/2$ p mm. With increasing depth of a reflector, the degree of concentration of the radiation is infact improved. However, this improvement will be smaller with a further increasing depth. Moreover, an increase of the depth of the reflectors will result in a corresponding increase of the dimensions of the housing. It has been found that with an increase of the depth of the reflectors above $3/2$ p mm a substantial improvement of the degree of concentration of the radiation is no longer obtained.

Advantageously, an irradiation apparatus according to the invention in a preferred embodiment is provided with a base which is hingedly connected by means of a telescopic arm to the housing accommodating the reflectors and by means of a hinge to the base of the apparatus, while the longitudinal axes of the base and of the housing lie substantially in the same plane and the base accommodates the ballast units for the high-pressure discharge lamps. The telescopic arm, which is provided with a hinge on either side, renders it possible, when the apparatus is not in use, to collapse and fold it and hence to considerably reduce its dimensions so that it occupies only little space. During operation of the apparatus, the telescopic arm permits of adjusting substantially arbitrarily the height and the direction of the housing, whilst the base, because it accommodates the ballast units, ensures the stability. Thus, it is superfluous to add to the base a separate mass as a mechanical stability ballast.

The base of the irradiation apparatus is preferably provided with a recess for receiving the telescopic arm in the collapsed state of the irradiation apparatus. Thus, a further reduction of the dimensions of the irradiation apparatus in the folded and collapsed state is obtained.

An irradiation apparatus according to the invention is preferably provided with high-pressure mercury vapour discharge lamps, the filling of which further contains cobalt and iron. The radiation emitted by this lamp in operation consists for 16 % of the overall power of UV-A radiation, whilst the contribution in the UV-B range (280 nm - 315 nm) and the dangerous UV-C range (200 nm - 280 nm) amounts to 3 % and 0.25 %, respectively. Thus, these lamps have the advantage that they emit a comparatively large quantity of UV-A radiation.

An irradiation apparatus according to the invention will be described more fully with reference to a drawing. In the drawing:

Fig. 1 is an elevation of the apparatus in the operational condition;

Fig. 2 is an elevation of the apparatus in the collapsed and folded state, and

Fig. 3 is a separate representation of the housing accommodating the reflectors and an associated field of irradiation.

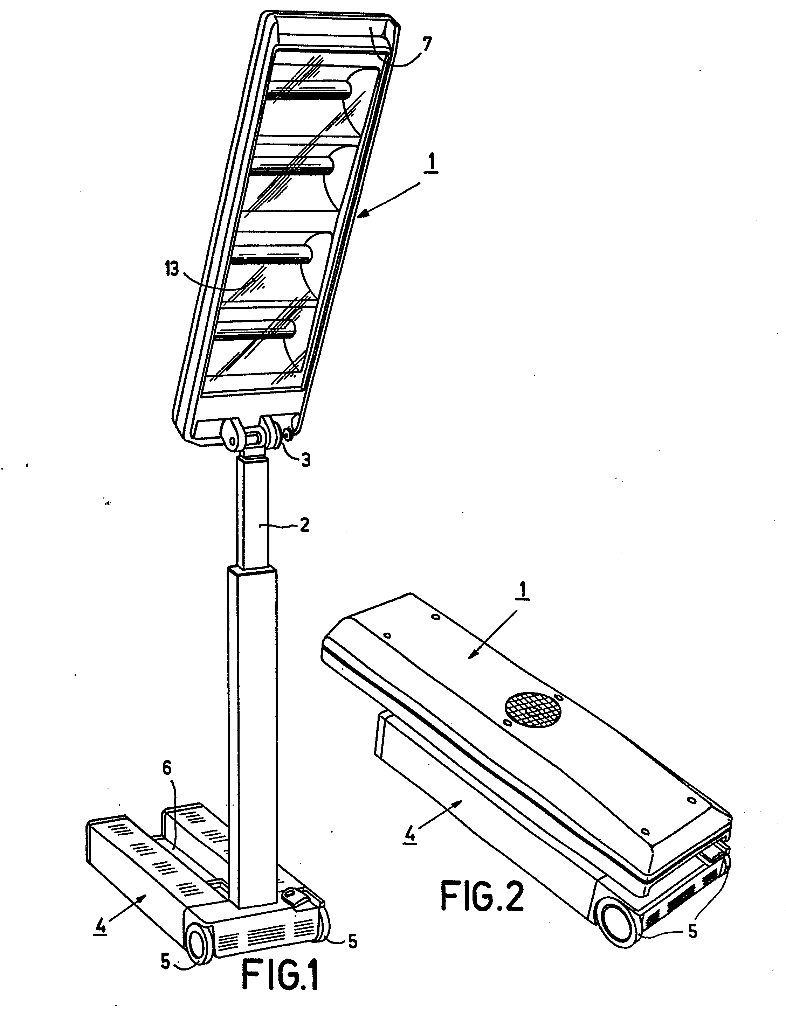

The housing 1 of Fig. 1 is connected by means of a hinge 3 to a telescopic arm 2 which at its other end is also provided with a hinge (not shown). The telescopic arm 2 connects the housing 1 to the base 4 of the irradiation apparatus. The base 4 is provided with two wheels 5. The base 4 is further provided with a recess 6 for receiving the telescopic arm 2 in the collapsed and folded state, as is shown in Fig. 2.

Fig. 1 further shows that the housing 1 is provided with a handle 7 so that by means of the handle the apparatus can be rolled on on the wheels 5, which favours the displaceability. The housing 1 is moreover provided with a filter 13 which is transparent and translucent to UV-A and which absorbs UV-B and UV-C radiation.

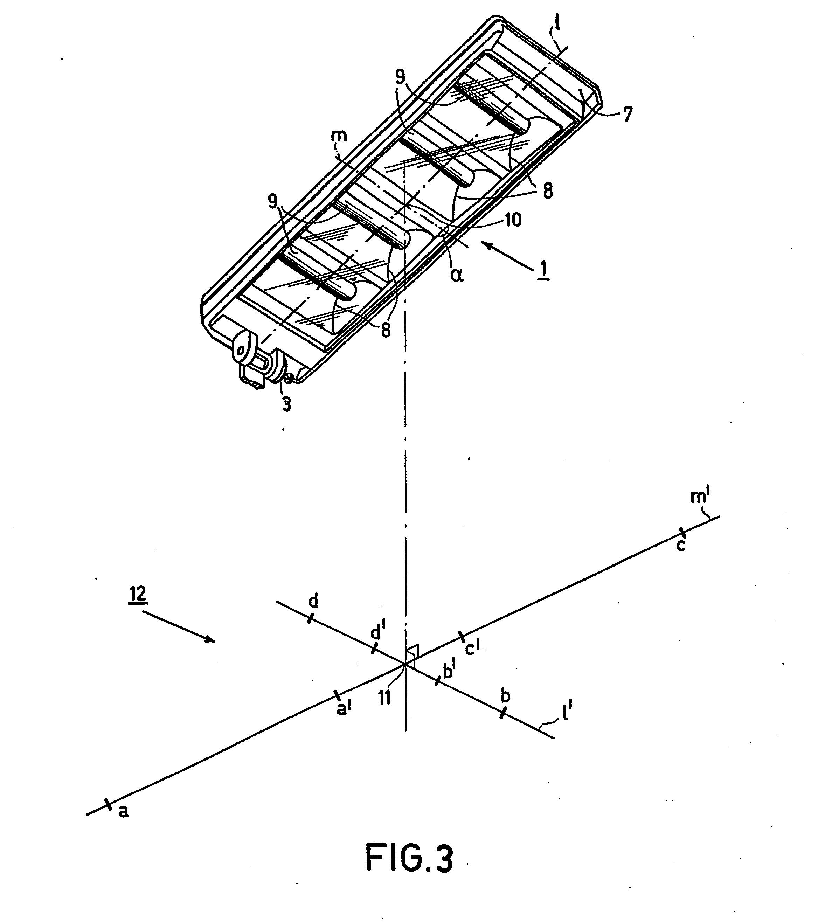

Fig. 3 separately shows the housing 1 and further a part of an associated field of irradiation 12. The longitudinal axis of the housing is designated by $l$ and
extends parallel to the axis of width $l'$ of the field of
irradiation.

The housing 1 comprises four identical reflectors
8 which are curved according to a parabola and whose focal
lines extend at a certain relative distance parallel to
each other. The reflectors 8 are subdivided into two groups
each of two reflectors so that each group has a common
alignment surface (not shown) and that the two directrix
planes have a line of intersection (not shown) parallel to
the line $\underline{m}$. The two directrix planes enclose  an obtuse
angle with each other, which corresponds to the obtuse
angle $\alpha$ between the respective emanation surfaces of the two
groups of reflectors. The line of intersection $\underline{m}$, which is
at right angles to the longitudinal axis $l$ , extends
parallel to the longitudinal axis m' of the field of
irradiation 12.

Each reflector is provided with one high-pressure
mercury vapour discharge lamp 9, the longitudinal axis of
the discharge vessel coinciding with a focal line of the
reflector and substantially coinciding with the discharge
path in the operational condition. The maximum length and
the maximum width of the field of irradiation 12 are
designated on the longitudinal axis m' and on the axis of
width $l'$, respectively, by the letters $\underline{a}$, $\underline{b}$, $\underline{c}$ and $\underline{d}$. The
point 11, which is the point of intersection of the two
axes and at the same time the perpendicular projection of
the centre 10 of the line of intersection $\underline{m}$, is the point
of maximum intensity. The letters a', b', c' and d'
designate the region around the point 11, in which the
radiation intensity is substantially equally as large as at
the point 11.

In a practical embodiment of the irradiation
apparatus, the housing has external dimensions of 95 cm x
30 cm x 11 cm. The housing accommodates four reflectors
which each are curved according to a parabola with a para-
meter $\underline{p}$ = 48 mm and have a depth of 58 mm. The obtuse angle
$\alpha$ enclosed by the two directrix planes is 174$^{\circ}$ on the side

facing the focal lines.

Each reflector has a length of 25 cm and is suitable for receiving a high-pressure discharge lamp having an external length of 13 cm and a power of 400 W.

The housing has a mass of 6 kg exclusive of lamps.

The base 4 of the apparatus is provided with four stabilization ballasts having an overall impedance of 180 $\Omega$. The maximum dimensions of the base are 74 cm x 33 cm x 12 cm and the mass is 28 kg.

The telescopic arm, which connects the base and the housing to each other, has a maximum length of 1.40 m.

In the collapsed and folded state, the maximum dimensions of the irradiation apparatus are 96 cm x 33 cm x 23 cm. The overall mass, inclusive of lamps, is 36 kg.

In the operational condition of the irradiation apparatus, in which a high-pressure mercury vapour discharge lamp having a dissipation power of 400 W is included in each reflector, the filling of each lamp contains besides 20 mg of mercury also 0.16 mg of cobalt and 0.3 mg of iron. The spectrum of the radiation emitted by the lamps comprises besides infrared radiation mainly UV-A and UV-B radiation. 16 % of the overall power dissipated in the lamp is UV-A radiation and only 3 % UV-B radiation.

With an irradiation at a surface parallel to both the focal lines of the parabolae and the longitudinal axis of the housing at a distance between the irradiated surface and the nearest discharge lamp of 1 m, the irradiation field has the following dimensions. The point of maximum intensity of the field of irradiation is designated in Fig. 3 by 11. The points a, b, c and d having an intensity half that of the point 11 are located at 90 cm, 90 cm, 32 cm and 32 cm, respectively, from the point 11, whilst the points a', b', c' and d' having the same intensity as the point 11 are located at 20 cm, 20 cm, 10 cm and 10 cm, respectively, from the point 11.

The dimensions of the field of irradiation are suitable for carrying out a total body irradiation. It is alternatively possible to carry out an irradiation of a

0106395
15-2-1983

part of the body, for example, the upper part of the body. The dimensions of the field of irradiation then render it possible for two persons at a time to be subjected to such an irradiation. Since the position of the housing of the irradiation apparatus is substantially arbitrarily adjustable, such an irradiation can be carried out on persons in a lying posture, but also in a sitting posture.

## CLAIMS

1.      An irradiation apparatus suitable for irradiation of the human body and provided with a housing comprising two or more elongate reflectors which are curved according to a parabola with a parameter $p$, whose focal lines extend at a certain relative distance parallel to each other and whose alignment surfaces enclose obtuse angles of at least $160^{\circ}$ and at most $200^{\circ}$ with each other, whilst each reflector is provided with one or more lamp holders suitable for receiving a high-pressure discharge lamp with the discharge path being substantially along the focal line, characterized in that each reflector is suitable for receiving only one lamp and in that for each reflector the parameter $p$ lies between the limits of 70 mm and 30 mm and the depth of each reflector is at least $p$ mm.

2.      An irradiation apparatus as claimed in Claim 1, characterized in that the reflectors are subdivided into two groups so that each group has a common alignment surface and that the two alignment surfaces on the sides facing the focal lines enclose an obtuse angle of at least $165^{\circ}$ and at most $180^{\circ}$ with each other.

3.      An irradiation apparatus as claimed in Claim 1 or 2, characterized in that the depth of each reflector is at most $3/2 \; p$ mm.

4.      An irradiation apparatus as claimed in Claim 1, 2 or 3, which is provided with a base which is hingedly connected by means of a telescopic arm to the housing accommodating the reflectors, characterized in that the telescopic arm is connected by means of a hinge to the base of the apparatus, in that the longitudinal axes of the housing and of the base lie substantially in the same plane and in that the base accommodates the ballast units for the high-pressure discharge lamps.

5.      An irradiation apparatus as claimed in Claim 4, characterized in that the base is provided with a recess for receiving the telescopic arm in the collapsed state of the irradiation apparatus.

6. An irradiation apparatus as claimed in Claim 1, 2, 3, 4 or 5, characterized in that the apparatus is provided with high-pressure mercury vapour discharge lamps, the filling of which further contains cobalt and iron.

FIG.1

FIG.2

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | DE-A-2 804 228 (WOLFF)<br>* Page 4, paragraph 1; page 7, paragraph 1; page 9, paragraph 1; figures 2,5,12 *<br><br>--- | 1,2 | A 61 N    5/06<br>F 21 S    3/12 |
| Y | EP-A-0 027 187 (SELLMEIER)<br>* Page 11, lines 17-23; page 13, lines 8-14; figure 5 *<br><br>--- | 1,4 | |
| A | GB-A-2 009 384 (SUN)<br>* Page 1, lines 115-121; page 3, lines 35-54; figures 3,10 *<br><br>--- | 1,3 | |
| A | DE-U-7 700 983 (WOLFF)<br>* Claim 1 *<br><br>--- | 1,2 | |
| A | DE-U-7 623 367<br>(PATENT-TREUHAND-GESELLSCHAFT)<br>* Claim 1 *<br><br>--- | 1,6 | **TECHNICAL FIELDS SEARCHED (Int. Cl. ³)** |
| A | US-A-3 310 673 (FLETCHER)<br>* Column 2, lines 28-41; figures 2,4 *<br><br>--- | 4,5 | A 61 N<br>F 21 V<br>F 21 S |
| A | FR-A-1 228 994 (BENTOLILA)<br>* Page 1, right-hand column; figures 1,2 *<br><br>---          -/- | 4,5 | |

The present search report has been drawn up for all claims

| Place of search<br>THE HAGUE | Date of completion of the search<br>19-12-1983 | Examiner<br>SIMON J.J.E. |
|---|---|---|

EPO Form 1503. 03.82

## European Patent Office

| Category | Citation of document with indication, where appropriate, of relevant passages | Relevant to claim | CLASSIFICATION OF THE APPLICATION (Int. Cl. ³) |
|---|---|---|---|
| A | FR-A-2 389 229 (PATENT-TREUHAND-GESELLSCHAFT) * Page 2, lines 5-19 * | 6 | |
| A | US-A-4 095 113 (WOLFF) | | |

### DOCUMENTS CONSIDERED TO BE RELEVANT

Page 2

TECHNICAL FIELDS SEARCHED (Int. Cl. ³)

The present search report has been drawn up for all claims

| Place of search | Date of completion of the search | Examiner |
|---|---|---|
| THE HAGUE | 19-12-1983 | SIMON J.J.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503, 03.82